# EUROPEAN PATENT APPLICATION

(11) **EP 4 703 935 A1**
(43) Date of publication of application: **04.03.2026**
(21) Application number: 25197846.6
(22) Date of filing: 25.08.2025
(51) Int. Cl.: G06F 21/57, G06N 20/00, G06Q 10/0635, G06Q 10/0637, G16H 50/30, H04L 9/40

(54) **SYSTEM FOR CYBER RISKS EVALUATION**

(30) Priority: 28.08.2024 US 202463687789 P
(71) Applicant: Levy, Gil, 6937908 Tel Aviv (IL); Peleg, Alexander, 7763222 Ashdod (IL); Mishalov, Michael, 9779331 Jerusalem (IL); Segal, Liran, 8101311 Yavne (IL); Gvozdenko, Andrey, 7528282 Rishon LeZion (IL)
(72) Inventor: Levy, Gil, 6937908 Tel Aviv (IL); Peleg, Alexander, 7763222 Ashdod (IL); Mishalov, Michael, 9779331 Jerusalem (IL); Segal, Liran, 8101311 Yavne (IL); Gvozdenko, Andrey, 7528282 Rishon LeZion (IL)
(74) Representative: Caldon, Giuliano

(57) **Abstract**

A method and system for evaluating cyber risk of an entity comprising a risk evaluation module configured to collect risk data on risks of cyber-attacks connected to SaaS, infrastructure, and legal regulations classified by geolocation, industry type, and size of the victim organization, an entity evaluation module for collecting vulnerability data on assets of the entity classified by industry type, geolocation, size and cyber threat vector vulnerabilities and a monetization engine configured to make an assessment of expected financial loss from a specified cyber-attack to an entity classified by geolocation, industry type, and size, based on the risk data.

## Description

### RELATED APPLICATIONS

This application claims the benefit of priority under 35 USC §119(e) of U.S. Provisional Patent Application No. 63/687,789 filed on 28 August 2024, the contents of which are incorporated herein by reference in their entirety.

### FIELD AND BACKGROUND OF THE INVENTION

The present invention, in some embodiments thereof, relates to a cybersecurity system, and, more particularly, but not exclusively, to recognizing factors, cataloguing information and developing a process to monetize cyber risks to an entity.

Cybercrime damages have been estimated to reach $6 trillion in 2021 and may be expected to rise to $10.5 trillion by 2025 with a 15% YoY growth. Many organizations' bookings do not capture Cyber risks like other risks. When an organization faces a cyber-attack, they are often unprepared financially. Many insurance companies provide cyber insurance underwriting based on high-level risk assessment but lack true monetization of these risks. In many cases, cyber risks are not properly evaluated during merger and acquisition processes (M&A), valuations, initial public offering (IPO), and as a financial key performance indicator (KPI).

CN Patent application No. 114757594 appears to disclose, "a method, a device, a terminal and a medium for monetizing network security risks. According to the scheme, firstly, a risk scene of a target enterprise is established, collected enterprise operation data and enterprise asset information are combined, an asset main body related to the risk scene and risk data related to the asset main body are determined, then the asset main body, the risk data, and protection level maturity scoring data are compared with obtained industry reference data, risk quantitative data and risk monetization data of the asset main body in the risk scene are determined, risk simulation is carried out in a risk simulation mode, and risk loss generated in a simulation result is converted into monetary property loss according to the risk quantitative data and the risk monetization quantitative data."

US Patent no. 11050778 appears to disclose, "An apparatus and method for cyber risk quantification calculated from the likelihood of a cyber-attack on the target enterprise and/or cyber ecosystem based on its security posture. The cyber-attack likelihood can be derived as a probability-based time-to-event (TTE) measure using survivor function analysis. The likelihood probability measure can also be passed to cyber risk frameworks to determine financial impacts of the cyber-attacks. Embodiments of the present invention also relate to an apparatus and method (1) to identify and validate application attack surfaces and protect web applications against business logic-based attacks, sensitive data leakage and privilege escalation attacks; and/or (2) that protects web applications against business logic-based attacks, sensitive data leakage and privilege escalation attacks. This can include implementing an intelligent learning loop using artificial intelligence that creates an ontology-based knowledge base from application request and response sequences. Stochastic probabilistic measures are preferably applied to a knowledge base for predicting malicious user actions in real time."

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
Fig. 1A-1D: Flow and block diagrams illustrating cyber risk monetization processes and systems, according to some embodiments.
Fig. 2A: Block diagram of various data sources in accordance with some embodiments.
Fig. 2B: Block diagram of the cyber risk monetization process in accordance with some embodiments.
Fig. 3: Flow diagram of the cyber risk monetization process in accordance with some embodiments.
Fig. 4: Flow diagram of a cyber risk assessment, in accordance with some embodiments.
Fig. 5: Flow diagram of a cyber risk assessment, in accordance with some embodiments.
Fig. 6: Schematic diagram illustrating cyber risk assessment dashboard output interface in accordance with some embodiments.
Figs. 7A and 7B: Schematic diagram illustrating cyber risk assessment risk score output interface in accordance with some embodiments.
Fig. 8: Schematic diagram illustrating a database in accordance with some embodiments.
Fig. 9 is a flow diagram of a cyber risk assessment product process flow, in accordance with some embodiments.
Fig. 10: Flow diagram of a cyber risk assessment, in accordance with some embodiments.

### SUMMARY OF THE INVENTION

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

In one general aspect, system may include a risk evaluation module for collecting risk data on cyber-attacks from multiple sources including SaaS platforms, infrastructure telemetry, and regulatory data feeds. System may also include an entity evaluation module for identifying vulnerability data on assets of the entity classified by industry type, geolocation, size and cyber threat vector vulnerabilities. System may furthermore include a structured data classification module that organizes the collected risk data by geolocation, industry type, and size of a victim organization. System may in addition include a trained machine learning monetization engine configured for: receiving the classified risk data, receiving the vulnerability data, correlating the risk data and the vulnerability data; correlating the classified risk data with historical cyber incident datasets and insurance claims and generates a probability-adjusted cyber incident profile having estimated financial loss values and incident likelihoods for an entity. Other embodiments of this aspect include corresponding computer systems, apparatus, and computer programs recorded on one or more computer storage devices, each configured to perform the actions of the methods.

Implementations may include one or more of the following features. System where said software module includes cyber agent executable on a cloud-based computing infrastructure and configured to act autonomously. System may include a user-facing analytics engine having a dashboard interface configured to present the incident profile in visual, board-level decision support format. System 2, 3, 4, 5, 6,7 and 8, where the risk data is collected, at least in part, over the cloud. System where the structured data classification module and monetization engine are configured to update assessments dynamically in response to new risk data supplied by the software module. System may include: an AI threat assessment module configured to estimate a probability of a specified cyber-attack on the entity based on said identified threats, and said identified vulnerabilities. System where said AI threat assessment module is further configured to estimate an impact of said specified cyber-attack based said risk data. System where said multiple sources include insurance claims. System where the risk data is further classified by demographics of involved populations. System where said involved populations include at least one of customers and employees. System where the monetization engine is configured to receive input data including customer data, system data and model data. System where the customer data includes at least one of organization data, Top-Level Domain (TLD) data, security control data, Data Loss Prevention (DLP) data, endpoint detection and response (EDR) data, email gateways, disaster recovery site (DR site), certificates, and asset contextualization. System where the model data is used to train the monetization engine. System where the system data includes at least one of organization meta data, public information, required regulations, baseline data, asset discovery, vulnerabilities, Cloud data, employee leaked credentials, threat intelligence insights, top advanced persistent threat (APT) profiles, preferred methods, geolocation and industry, brand protection, phishing sites, data leaks from prior attacks. System where the model data includes at least one of industry cataloging, geolocation cataloging, insurance claim, root cause, data privacy violations fines, or historical events. System where the financial loss values and incident likelihoods are from at least one of subdomain hijacking, email attacks, network security, DNS configuration, cloud security, data breach, social engineering, webside application firewall (WAF) configuration, vulnerable technologies, service security or a combination thereof. System where the social engineering includes identification of employees, password leaks, or phishing. Implementations of the described techniques may include hardware, a method or process, or a computer tangible medium.

**In** one general aspect, computer - implemented method may include collecting cyber risk data from at least one of a SaaS system, an infrastructure log, a legal database and a regulatory database. Computer - implemented method may also include classifying the risk data by location, industry type, and size of a victim organization. The method may furthermore include identifying vulnerabilities for the entity. The method may in addition include estimating a probability of a specified cyber-attack by correlating said risk data and said vulnerabilities. Other embodiments of this aspect include corresponding computer systems, apparatus, and computer programs recorded on one or more computer storage devices, each configured to perform the actions of the methods.

Implementations may include one or more of the following features. The method may include executing a predictive monetization model to calculate an expected financial loss from a specified cyber-attack to an entity classified by location, industry type, and size based on the collected risk data using a predictive monetization engine. The method may include: training the predictive monetization model using: historical cyber insurance claim data; previously reported vulnerability disclosures; and confirmed threat intelligence indicators. The method may include: presenting an assessment of a plurality of said expected financial losses to at least one of stakeholders, boards, and auditors using a visual dashboard and reporting module. The method may include: Automatically retraining the predictive monetization model periodically using: historical cyber insurance claim data; previously reported vulnerability disclosures; and confirmed threat intelligence indicators. The method may include: estimating an impact of said specified cyber-attack based on said identified threats and an identified vulnerabilities using the predictive monetization model. The method may include: collecting said risk data from insurance claims of reported threats and reported vulnerabilities. The method where said classifying is further by demographics of involved populations. The method where said involved populations include at least one of customers and employees. The method where said collecting is at least in part, over an Internet cloud. The method where the predictive monetization model receives input data including customer data, system data and model data. The method where the customer data includes at least one of organization data, Top-Level Domain (TLD) data, security control data, Data Loss Prevention (DLP) data, endpoint detection and response (EDR) data, email gateways, disaster recovery site (DR site), certificates, and asset contextualization. The method where the system data includes at least one of organization meta data, public information, required regulations, baseline data, asset discovery, vulnerabilities, Cloud data, employee leaked credentials, threat intelligence insights, top advanced persistent threat (APT) profiles, preferred methods, geolocation and industry, brand protection, phishing sites, data leaks from prior attacks. The method where the model data includes at least one of industry cataloging, geolocation cataloging, insurance claim, root cause, data privacy violations fines, or historical events. The method where the risks include subdomain hijacking, email attacks, network security, DNS configuration, cloud security, data breach, social engineering, webside application firewall (WAF) configuration, vulnerable technologies, service security or a combination thereof. The method where the social engineering includes identification of employees, password leaks, or phishing. Implementations of the described techniques may include hardware, a method or process, or a computer tangible medium.

### DESCRIPTION OF SPECIFIC EMBODIMENTS OF THE INVENTION

The present invention, in some embodiments thereof, relates to a cybersecurity system, and, more particularly, but not exclusively, to recognizing factors, cataloging information and developing a process to monetize cyber risks to an entity.

### OVERVIEW

According to some embodiments, the cybersecurity system may employ various technologies and/or systems to discover and/or monetize vulnerabilities, advise on the allocation of resources for remediating the discovered vulnerabilities, ensure that the company complies with national and/or international regulations and/or provide security certifications. In some embodiments, discovery of risks may be supplied from an external source.

According to some embodiments, the cybersecurity system may use various technologies to identify a company's cybersecurity vulnerabilities e.g., using discovery engines and/or algorithms, such as identifying websites, infrastructure and/or leaked data. Optionally, the cybersecurity system may identify externally exposed cloud, web, mobile, and/or infrastructure assets. Optionally, the cybersecurity system may identify leaks of a company's and/or employees' sensitive data. Optionally, the cybersecurity system may provide a view of the threats against a company. Optionally, such identification may be performed continuously. In some embodiments, identification of exposed assets may be supplied from an external source.

Some embodiments may relate to transforming cyber exposure into measurable, actionable financial insights. Advantageously, according to some embodiments, the system may provide outstanding values by helping financial officer understand their organization's exposure and/or monetize the risks so they can prioritize protection, factor them into their bookings, help insurance companies understands their customer's cyber risk in monetary terms and provide accurate underwriting, and help to factor in cybersecurity risks in company evaluations in the event of mergers, acquisitions, IPO, company valuations, credit ranking, business to business loans and/or credit lines, etc.

Additionally, according to some embodiments, such a system may advantageously provide visibility to a company's business operating division on their existing cyber risks, provide cyber risk assessments for insurance, credit ranking, valuations for mergers, acquisitions, IPO, etc. and/or risk audit.

According to some embodiments, organizations in the public and/or private sectors may find it challenging to prioritize limited resources toward remediating the vulnerabilities that are most likely to result in a damaging intrusion. Optionally, it may be difficult for a company to assess where digital assets may be exposed and/or used to attack. Optionally, growing companies may be unaware of requirements to comply with national and/or international regulations, such as monitoring potentially exposed assets, and receiving security certifications.

According to some embodiments, input data may include customer data and/or system data and/or model data. A system may include Data Loss Protection (DPL). According to some embodiments, customer data may include the organization or Top-Level Domain (TLD) to categorize websites and/or assist in guiding requests to the correct destination. Additionally, or alternatively, customer data may include security control data e.g., DPL data. For example, DPL may include cybersecurity that detects and prevents data breaches, e.g., by blocking extraction of sensitive. Optionally, DPL may include internal security and/or regulatory compliance; endpoint detection and response (EDR) and/or extended detection and response (XDR). The system may include cybersecurity solutions that detect, investigate, and respond to security incidents; email gateways; disaster recovery site (DR site) which may include a secondary location used by an organization to recover and restore its IT infrastructure and operations when the primary data center is unavailable due to a disaster or other disruption; certificates; assets contextualization (e.g., pending discovery); or a combination thereof.

According to some embodiments, system data may include organization meta data, such as headquarters location, whether the organization is private or public, public information (e.g., size, revenue, etc.), required regulations (e.g., data privacy), baseline (e.g., similar organizations, industry, geolocation, size, etc.). Optionally, system data may include asset discovery, such as any internet-facing asset associated with the organization; vulnerabilities (e.g., IT infrastructure); Cloud data (e.g., pending customer consent); employee leaked credentials; threat intelligence insights (top advanced persistent threat (APT) profiles, which are sophisticated, sustained cyberattacks in which an intruder establishes an undetected presence in a network in order to steal sensitive data over a prolonged period of time; preferred methods, geolocation and industry; brand protection (e.g., information theft, etc.); phishing sites (e.g., twister domains wherein attackers register domains with small spelling changes, like swapping letters or adding extra characters, to trick users into visiting malicious sites or responding to emails they believe are from someone they know); data leaks from prior attacks; or a combination thereof.

According to some embodiments, model data may include industry cataloging; geolocation cataloging; insurance claim (e.g., settlement, penalty, etc.); root cause (e.g., attack type, breach type, etc.); data privacy violations fines; historical events; or a combination thereof. Optionally, the model data may be used to train the AI model. Non-limiting examples of types of risk may include: subdomain hijacking; email attacks; network security (e.g., DNS configuration, etc.); cloud security (e.g., instances, storage, etc.); data breach (e.g., leaks, etc.); social engineering (e.g., identification and/or password leaks, phishing, etc.); webside application firewall (WAF) configuration; vulnerable technologies; service security (e.g., exposed service ports, etc.); or a combination thereof.

According to some embodiments, risk data may include data on risks of cyber-attacks connected to SaaS, infrastructure, and legal regulations classified by geolocation, industry type, and size of the victim organization, etc. Optionally, risk data may be collected from insurance claims, reported threats, reported vulnerabilities, etc. Optionally, the risk data may be further classified by demographics of involved populations, such as customers and/or employees.

According to some embodiments, the cybersecurity system may validate the risk, e.g., run validation engines and/or algorithms to understand where an attack may come from. In some embodiments, the validation aims to reduce the false positive cases and/or enrich the discovery results with additional information on the vulnerability and/or to detect additional issues Optionally, the system continuously updates such engines and/or algorithms as trending exploitable vulnerabilities, etc. Optionally, the validation engines may be automated. In some embodiments, validation reduces the false positive cases and/or enriches the discovery results with additional information on the vulnerability and/or by detecting additional issues

According to some embodiments, the cybersecurity system may assess the possibility to prevent a breach in cyber security. Optionally, the reduction to risk by preventing a cybersecurity breach may be analyzed and monetized by an automated monetization engine and/or algorithm. Optionally, recommendations may include ways to reduce indemnity, for example, by added protective elements, for example, a cybersecurity breach may be prevented by a person of skill in the field based on the cybersecurity system. Optionally, the risk of breach and/or ransomware may be reduced by continuously validating a company's assets, e.g., against the latest trending exploits.

According to some embodiments, the system may include a risk monetarization calculator for cyber-risk. Optionally, the system may identify specific cyber risks e.g., known attack methodologies, weaknesses in software, weaknesses in infrastructure, etc. Optionally, the system may categorize likely types of attacks. Optionally, the model may determine the expected risks and/or costs of each exposed risk. Optionally, the model may break down company characteristics and/or look at costs of past attacks on similar companies. Optionally, this may be included in the analysis and/or calculations. Optionally, the system may include a weighting system, e.g., the more similar a previous attack, the more its historical costs may weigh into the calculations.

According to some embodiments, some issues which may be taken into account in the cyber risk monetarization calculator may include data breaches, leaked credentials, exposed assets, cloud misconfiguration, supply chain breaches, etc. Each possibility is a separate embodiment. Optionally, each issue may be assigned a weighted value.

According to some embodiments, some risks which may be monetized in the cyber risk analysis of the risk monetarization calculator (e.g., monetization engine) may include ransomware, IoT attack, cloud attack, phishing attack, blockchain and cryptocurrency attacks, SW vulnerabilities, machine learning and AI attacks, stolen funds, loss of business continuity, etc. Each possibility is a separate embodiment. Optionally, each risk may be assigned a weighted value.

According to some embodiments, the type of industry which the company is in may be taken into account in the monetization of the cyber risk analysis of the risk monetarization calculator (e.g., monetization engine), such as healthcare, energy, financial, pharma, technology, industrial, services, entertainment, education, transportation, communication, consumer, retail, hospitality, media, research, etc. Each possibility is a separate embodiment. Optionally, each type of industry may be assigned a weighted value.

According to some embodiments, the geolocation of the company may be taken into account in the monetization of the cyber risk analysis of the risk monetarization calculator, such as North America, South America, Europa, Asia, Africa, Australia, virtual locations (metaverse), cloud, etc. Each possibility is a separate embodiment. Optionally, each geolocation may be assigned a weighted value.

According to some embodiments, monetization may include material costs, such as investigation, recovery, etc. Each possibility is a separate embodiment. Optionally, each material cost may be assigned a weighted value.

According to some embodiments, monetization may include non-material costs, such as data breaches, leaked credentials, exposed assets, cloud misconfiguration, supply chain breaches, etc. Each possibility is a separate embodiment. Optionally, each non-material cost may be assigned a weighted value.

### SPECIFIC EMBODIMENTS

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

The present invention, in some embodiments thereof, relates to a cybersecurity risk model for cyber risks monetization, and, more particularly, but not exclusively, to discover and mitigate cyber risks.

Reference is now made to the figures.

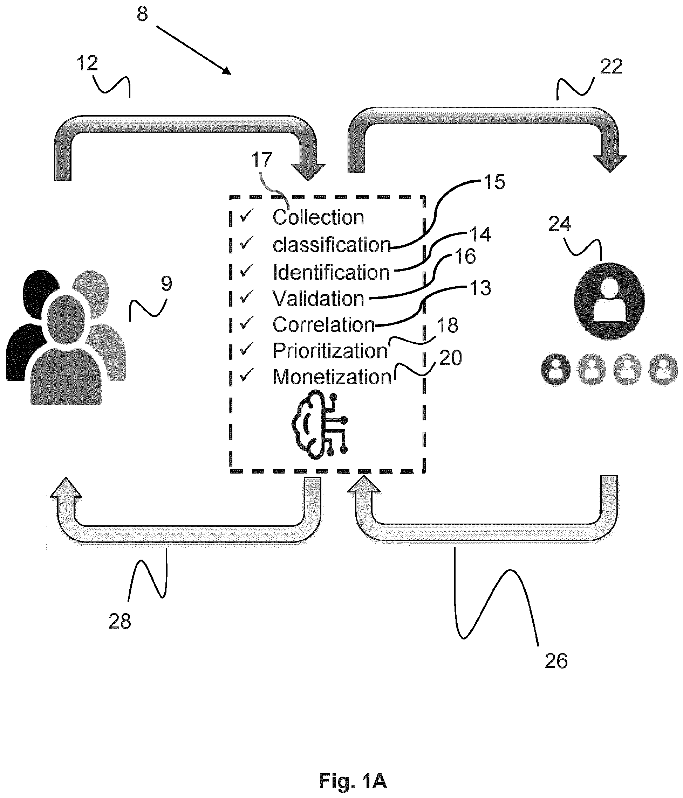

Fig. 1A is a flow diagram illustrating a cyber risk monitoring process, according to some embodiments. For example, in process 8, a software module (e.g., a cyber analyst 9) identifies vulnerability of the customer company and/or its assets using a process of identification14 of vulnerabilities of the company and collects 17 data on threats using a variety of input data to identify and monetized vulnerabilities 12 and threats. Optionally, the identification 14 process may identify specific cyber vulnerabilities e.g., known attack vectors, weaknesses in software, weaknesses in infrastructure, etc. Optionally, the system may classify 15 threats and/or vulnerabilities (for example, the industries and/or the geolocation of the company and/or its assets). Optionally, the model may correlate 13 the vulnerabilities to the classified threats. For example, the system may check if vectors identified 14 in the vulnerabilities of the company are methods of attack of threats in the industry and/or region of the company's assets. This will give, for example, probability of certain attacks and probability of success on various assets of the company from which the system may determine expected risks and/or costs. Optionally, the system may break down company characteristics and/or look at costs of past attacks on similar companies. Risk validation 16 may then be undertaken e.g., by running validation engines and/or algorithms to understand where an attack may come from. Optionally, the risk validation 16 may be continuously updated. Optionally, the risk validation 16 engines may be automated. Assessment and prioritization of high-risk vulnerabilities 28 e.g., for increased cybersecurity and/or protection. The determined vulnerabilities may be prioritized 18. The determined vulnerabilities may be monetized 20 by a monetization engine. Optionally, monetization 20 may include material costs, such as investigation, recovery, etc. Optionally, monetization 20 may include non-material costs, such as data breaches, leaked credentials, exposed assets, cloud misconfiguration, supply chain breaches, exposure to legal risks, etc. Optionally, the monetized vulnerability assessment 22 may be reported to the customer 24 with recommendations on how to prevent attacks and/or reduce the effect of an attack and/or protect the company and/or ensure compliance of the company with regulations and laws. Optionally, once the customer 24 has implemented the recommendations, the identification 14, validation 16, and prioritization 18 process may be repeated. Optionally, monetization 20 may be adjusted based on business impact 26, etc. Optionally, repeating the process may ensure that the monetization remains accurate. Optionally, the process may be repeated after a cyber attack on the company and/or similar company. Optionally, the process may be repeated daily, weekly, monthly, quarterly, yearly, etc.

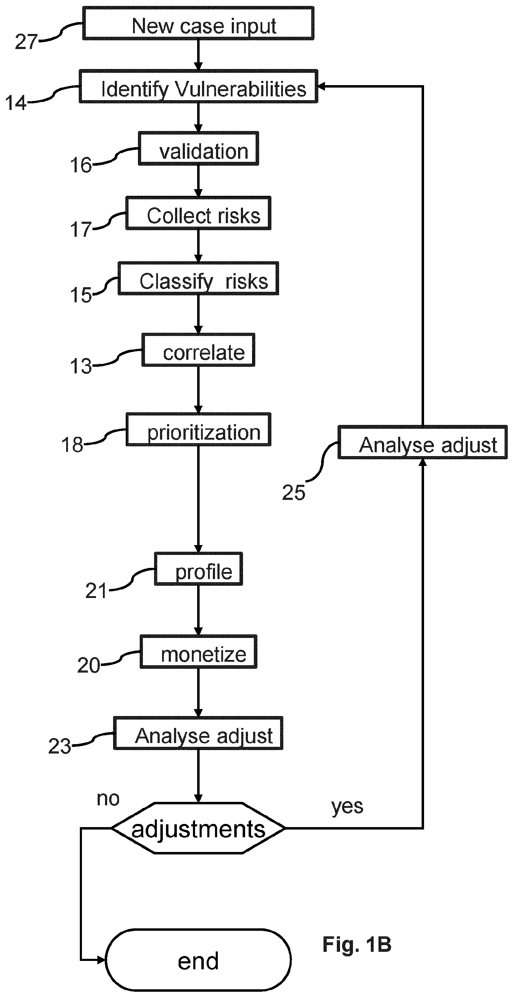

Fig. 1B is a flow diagram illustrating a cyber risk monitoring process, according to some embodiments. In some embodiments, an entity is inputs 27 data. Optionally, the vulnerabilities 12 of the entity are identified 14 using a variety of input data. Optionally, the identification 14 process may identify specific cyber risks e.g., known attack methodologies, weaknesses in software, weaknesses in infrastructure, etc. Risk validation 16 may then be undertaken e.g., by running validation engines and/or algorithms to understand whether the vulnerabilities are real. Optionally, the system may collect 17 information about risks of attacks (e.g., threats). For example, the system may include an independent AI agent that searches the web for current, predicted and historical data on cyber-attacks. Alternatively, data may be acquired from another source (e.g., bought) and/or collected by an external module and/or an external service. In some embodiments, the system classifies15 threats and/or vulnerabilities. Optionally, the model may determine expected risks and/or costs. Optionally, the system may break down company characteristics and/or look at costs of past attacks on similar companies. Optionally, the risk validation 16 engines may be automated. Assessment and prioritization of high-risk vulnerabilities e.g., for increased cybersecurity and/or protection. The determined vulnerabilities may be prioritized 18. Optionally, the system will account for a profile 21 of the entity. For example, the location of the entity and size and type of entity and/or its assets. In some embodiments, the profile may change the expected claims (e.g., in some countries lawyer costs and/or damage awards may be greater or smaller) maybe accounted for to adjust the expected costs from a claim and/or tbe likelihood of various types of attacks. The determined vulnerabilities may be monetized 20 by a monetization engine. For example, the system may evaluate the possible claims that may come from an attack (e.g., claims from customers, claims from other entities etc.). Optionally, monetization 20 may include material costs, such as investigation, recovery, etc. Optionally, monetization 20 may include non-material costs, such as data breaches, leaked credentials, exposed assets, cloud misconfiguration, supply chain breaches, exposure to legal risks, etc. Optionally, the monetized vulnerability assessment 22 may be analyzed and/or adjusted 23 (for example by a human analysist. Optionally, the identification 14, collection 17, classification 15, validation 16, correlation 13, prioritization 18 and/or monetization 20 may be continuously updated. The results are optionally reported to the customer with recommendations on how to prevent attacks and/or reduce the effect of an attack and/or protect the company and/or ensure compliance of the company with regulations and laws. Optionally, the customer implements the recommendations or recommends adjustments based on his knowledge of the entity. The new situation and/or data may be analyzed 25 and/or the identification 14, validation 16, and prioritization 18 process may be repeated. Optionally, monetization 20 may be adjusted based on business impact etc. Optionally, repeating the process may facilitate updating the monetization to remain accurate. Optionally, the process may be repeated in response to new external data e.g., a cyber-attack on the company and/or similar company. Optionally, the process may be repeated daily, weekly, monthly, quarterly, yearly, etc.

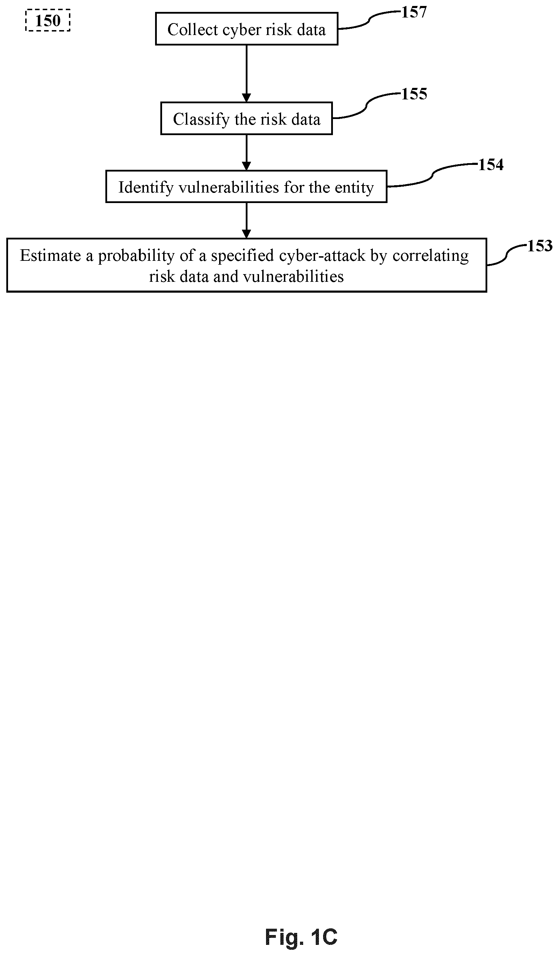

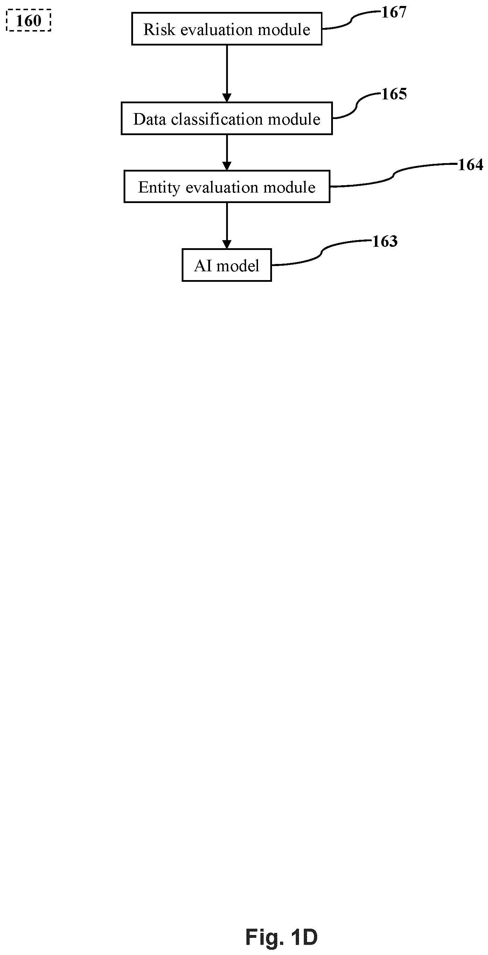

Fig. 1C is a flowchart of an example process 150. In some implementations, one or more process blocks of Fig. 1 may be performed by a device.

As shown in Fig. 1, process 150 may include collecting 157 cyber risk data. For example, the data may be collected 157 from a SaaS system, an infrastructure log, a legal database and a regulatory database. For example, data may be collected 157 by a software module (e.g., an independent agent). As also shown in Fig. 1C, process 150 may include classifying 155 the risk data (e.g., by location, industry type, and size of a victim organization). As further shown in Fig. 1, process 150 may include identifying 154 vulnerabilities for the entity. As also shown in Fig. 1, process 150 may include estimating 153 a probability of a specified cyber-attack. For example, probably may be estimated 153 by correlating risk data and vulnerabilities. For example, device may estimate a probability of a specified cyber-attack by correlating said risk data and said vulnerabilities.

Although Fig. 1C shows example blocks of process 150, in some implementations, process 150 may include additional blocks, fewer blocks, different blocks, or differently arranged blocks than those depicted in Fig. 1. Additionally, or alternatively, two or more of the blocks of process 150 may be performed in parallel.

In some embodiments, the method may include executing a predictive monetization model to estimate an expected financial loss from a specified cyber-attack to an entity classified 155 by location, industry type, and size based on the collected 157 risk data using a predictive monetization engine. This predictive monetization model may be designed to provide a financial estimate of the potential impact of cyber-attacks, allowing entities to better understand their exposure and prioritize their cybersecurity efforts accordingly.

Optionally, the method may further comprise training the predictive monetization model using historical cyber insurance claim data, previously reported vulnerability disclosures, and confirmed threat intelligence indicators. By leveraging these diverse data sources, the predictive monetization model can be fine-tuned to improve its accuracy and reliability in estimating financial losses.

In some embodiments, the method may include automatically retraining the predictive monetization model periodically using historical cyber insurance claim data, previously reported vulnerability disclosures, and confirmed threat intelligence indicators. This periodic retraining may facilitate keeping the model up-to-date. For example, the model is updated according to with the latest trends and threats in the cybersecurity landscape. Optionally, this may facilitate maintaining its effectiveness over time.

Additionally, the method may further comprise estimating an impact of the specified cyber-attack based on collected 157 threats and identified 154 vulnerabilities using the predictive monetization model. This step may allow for a more granular assessment of potential damages, taking into account the specific nature of the threats and vulnerabilities faced by the entity.

Optionally, the method may include collecting 157 risk data from insurance claims of reported threats and reported vulnerabilities. This data collection 157 step can provide valuable insights into real-world incidents and/or their consequences, enriching the dataset used to train and refine the predictive monetization model.

In some embodiments, the method may further classify 155 the risk data by demographics of involved populations, such as customers and employees. This classification 155 can help in understanding how different segments of the population might be affected by cyber-attacks, thereby enabling more targeted risk management strategies.

Optionally, the method may include presenting an assessment of a plurality of expected financial losses to stakeholders, boards, and auditors using a visual dashboard and reporting module. This presentation step facilitates clear communication of the potential financial impacts of cyber risks, aiding decision-makers in their risk management and mitigation efforts.

In some embodiments, the method may involve collecting 157 risk data at least in part over an Internet cloud. This approach can enhance the scalability and accessibility of the data collection 157 process, allowing for more comprehensive and timely data gathering.

The predictive monetization model may receive input data including customer data, system data, and model data. Customer data may include organization data, Top-Level Domain (TLD) data, security control data, Data Loss Prevention (DLP) data, endpoint detection and response (EDR) data, email gateways, disaster recovery site (DR site) certificates, and asset contextualization. These diverse data points provide a holistic view of the entity's cybersecurity posture.

System data may include organization metadata, public information, required regulations, baseline data, asset discovery, vulnerabilities, cloud data, employee leaked credentials, threat intelligence insights, top advanced persistent threat (APT) profiles, preferred methods, geolocation, industry brand protection, phishing sites, and data leaks from prior attacks. This comprehensive dataset enables a thorough assessment of the entity's cybersecurity environment.

Model data may include industry cataloging, geolocation cataloging, insurance claim root cause data, privacy violations, fines, and historical events. These data points help in contextualizing the risk data and enhancing the predictive capabilities of the monetization model.

The risks considered in the method may include subdomain hijacking, email attacks, network security, DNS configuration, cloud security, data breach, social engineering, website application firewall (WAF) configuration, vulnerable technologies, and service security. Each type of risk can be analyzed to understand its potential impact on the entity.

Social engineering risks may encompass identification of employees, password leaks, and phishing. By addressing these specific threats, the method can help in mitigating some of the most common and damaging cyber-attack vectors.

According to some embodiments, the cybersecurity system may employ various technologies and systems to discover and monetize vulnerabilities, advise on the allocation of resources for remediating the identified 154 vulnerabilities. The system may facilitate compliance with national and international regulations, and/or provide security certifications. Discovery of risks may be supplied from an external source, enhancing the system's ability to identify 154 vulnerabilities and/or collect 157 data on threats and/or address potential threats.

The system may use discovery engines and algorithms to identify 154 a company's cybersecurity vulnerabilities, such as websites, infrastructure, and leaked data. It may also inventory externally exposed cloud, web, mobile, and infrastructure assets, as well as leaks of sensitive data. The system may provide a continuous view of threats against a company, with identification 154 of exposed assets potentially supplied from an external source.

Transforming cyber exposure into measurable, actionable financial insights can provide significant value by helping financial officers understand their organization's exposure and monetize risks. This can aid in prioritizing protection measures, assisting insurance companies in underwriting, and factoring cybersecurity risks into company evaluations for mergers, acquisitions, IPOs, credit rankings, business loans, and credit lines.

The system may provide visibility into existing cyber risks for business operating divisions, offer cyber risk assessments for insurance and credit ranking valuations, and support risk audits. Public and private sector organizations may find it challenging to prioritize resources toward remediating the most likely damaging vulnerabilities, assess where digital assets may be exposed, and comply with national and international regulations.

Input data may include customer data, system data, and model data. Customer data may encompass organization or TLD data, security control data, DPL data, EDR, XDR, email gateways, DR sites, certificates, and asset contextualization. System data may include organization metadata, public information, required regulations, baseline data, asset discovery, vulnerabilities, cloud data, employee leaked credentials, threat intelligence insights, top APT profiles, preferred methods, geolocation, industry brand protection, phishing sites, and data leaks from prior attacks. Model data may include industry cataloging, geolocation cataloging, insurance claim root cause data, privacy violations, fines, and historical events.

Risk data may include data on cyber-attack risks connected to SaaS infrastructure and legal regulations, classified 155 by geolocation, industry type, and size of the victim organization. Risk data may be collected 157 from insurance claims, reported threats, and reported vulnerabilities, and further classified 155 by demographics of involved populations, such as customers and employees.

The cybersecurity system may validate risks using validation engines and algorithms to understand potential attack vectors, reduce false positives, enrich discovery results, and detect additional issues. Validation engines may be automated and continuously updated to address trending exploitable vulnerabilities.

The system may assess the possibility of preventing cybersecurity breaches, with risk reduction analyzed and monetized by an automated monetization engine. Recommendations may include ways to reduce indemnity, such as adding protective elements, and continuously validating company assets against the latest exploits.

The system may include a risk monetization calculator for cyber-risk, discovery of specific cyber risks, categorizing likely attack types, and determining expected risks and costs. The model may break down company characteristics and incorporate costs of past attacks on similar companies, with a weighting system for historical costs.

Issues considered in the cyber risk monetization calculator may include data breaches, leaked credentials, exposed assets, cloud misconfiguration, and supply chain breaches. Risks monetized may include ransomware, IoT attacks, cloud attacks, phishing attacks, blockchain and cryptocurrency attacks, software vulnerabilities, machine learning and AI attacks, stolen funds, and loss of business continuity.

The type of industry and geolocation of the company may be considered in the monetization, with weighted values assigned to each. Material costs, such as investigation and recovery, and non-material costs, such as data breaches and leaked credentials, may also be assigned weighted values.

Fig. 1D is a block diagram of a system 160 for evaluating cyber risk of an entity in accordance with an embodiment of the current invention. In some embodiments, the system may include several key modules and components that interact to assess and predict potential cyber threats and their financial implications. The system may be designed to gather, classify, and analyze data from various sources to provide a comprehensive risk evaluation.

The system may include a risk evaluation module 167 for collecting risk data on cyber-attacks from multiple sources. These sources can include SaaS platforms, infrastructure telemetry, and regulatory data feeds. The risk evaluation module 167 may be configured to aggregate this data to provide a broad understanding of the current cyber threat landscape.

Additionally, the system may feature an entity evaluation module 164 for identifying vulnerability data on assets of the entity. This module 164 may classify the assets by industry type, geolocation, size, and cyber threat vector vulnerabilities. By organizing the vulnerability data in this manner, the entity evaluation module 164 can help pinpoint specific weaknesses that are pertinent to the entity's particular circumstances.

A data classification module 165 may be included to organize the collected risk data by geolocation, industry type, and size of a victim organization. This module 165 may ensure that the risk data is systematically categorized, facilitating more accurate and relevant analysis.

The system may further comprise a trained machine learning monetization engine 163, such as an AI model, which is configured to perform several critical functions. This engine 163 may receive the classified risk data and the vulnerability data, and then correlate these datasets. Additionally or alternatively, the machine learning monetization engine 163 may correlate the classified risk data with historical cyber incident datasets and insurance claims. Through these correlations, the engine 163 can generate a probability-adjusted cyber incident profile. This profile may include estimated financial loss values and incident likelihoods for the entity, providing a detailed prediction of potential cyber risks and their economic impact.

In some embodiments, the system 160 for evaluating cyber risk of an entity may integrate multiple modules, including a risk evaluation module 167, an entity evaluation module 164, a data classification module 165, and/or a trained machine learning monetization engine 163. These components may work together to collect, classify, and analyze data, ultimately generating a comprehensive cyber risk assessment for the entity.

In some embodiments, the risk evaluation module 167 may be configured to assess various risks associated with cybersecurity threats. In some embodiments, the risk evaluation module may dynamically update assessments in response to new risk data supplied by a software module, which may include a cyber agent executable on a cloud-based computing infrastructure. The cyber agent may be configured to autonomously discover vulnerabilities, advise on the allocation of resources for remediating discovered vulnerabilities, ensure compliance with national and/or international regulations, and provide security certifications. Optionally, discovery of risks may be supplied from an external source.

In some embodiments, the entity evaluation module 164 may be configured to evaluate the cybersecurity posture of an entity by identifying vulnerabilities using discovery engines and algorithms. For example, the entity evaluation module may identify websites, infrastructure, and leaked data. Optionally, the module may identify externally exposed cloud, web, mobile, and infrastructure assets, as well as leaks of sensitive data belonging to the company and/or its employees. In some embodiments, identification of exposed assets may be performed continuously and may be supplied from an external source.

In some embodiments, a machine learning engine (e.g., module 163) may be configured to transform cyber exposure into measurable, actionable financial insights. Advantageously, the machine learning engine may help financial officers understand their organization's exposure and monetize the risks to prioritize protection, factor them into bookings, assist insurance companies in understanding their customer's cyber risk in monetary terms, provide accurate underwriting, and factor cybersecurity risks into company evaluations for mergers, acquisitions, IPOs, credit rankings, business-to-business loans, and credit lines. Additionally, the machine learning engine may provide visibility to a company's business operating division on existing cyber risks, offer cyber risk assessments for insurance, credit ranking, valuations for mergers, acquisitions, IPOs, and risk audits.

In some embodiments, the structured data classification module 165 may be configured to classify risk data, which may include data on risks of cyber-attacks connected to SaaS infrastructure and legal regulations, classified by geolocation, industry type, and size of the victim organization. In some embodiments, the monetization engine 163 may be configured to receive input data including customer data, system data, and model data. Customer data may include organization data, Top-Level Domain (TLD) data, security control data, Data Loss Prevention (DLP) data, endpoint detection and response (EDR) data, email gateways, disaster recovery site (DR site) certificates, and asset contextualization. System data may include organization metadata, public information, required regulations, baseline data, asset discovery, vulnerabilities, cloud data, employee leaked credentials, threat intelligence insights, top advanced persistent threat (APT) profiles, preferred methods, geolocation, industry brand protection, phishing sites, data leaks from prior attacks, or a combination thereof. Model data may include industry cataloging, geolocation cataloging, insurance claim root cause data, privacy violations, fines, historical events, or a combination thereof. Optionally, the model data may be used to train the monetization engine.

In some embodiments, the system 160 may further comprise an AI threat assessment module configured to estimate the probability of a specified cyber-attack on the entity based on identified threats and vulnerabilities. Optionally, the AI threat assessment module may also estimate the impact of the specified cyber-attack based on risk data. The risk data may be collected from multiple sources, including insurance claims, and may be further classified by demographics of involved populations, such as customers and employees.

Additionally or alternatively, the system 160 may include a user-facing analytics engine comprising a dashboard interface configured to present the incident profile in a visual board-level decision support format. Optionally, the risk data may be collected at least in part over the cloud.

In some embodiments, the financial loss values and incident likelihoods may be derived from various types of cyber risks, including subdomain hijacking, email attacks, network security, DNS configuration, cloud security, data breach, social engineering, website application firewall (WAF) configuration, vulnerable technologies, service security, or a combination thereof. Social engineering risks may include identification of employees, password leaks, or phishing.

The cybersecurity system may validate risks by running validation engines and algorithms to understand where an attack may come from, aiming to reduce false positive cases and enrich discovery results with additional information on vulnerabilities. Optionally, the validation engines may be automated and continuously updated.

The system may assess the possibility of preventing cybersecurity breaches and may analyze and monetize the reduction of risk by preventing such breaches. Recommendations may include ways to reduce indemnity by adding protective elements. Optionally, the risk of breach and ransomware may be reduced by continuously validating a company's assets against the latest trending exploits.

According to some embodiments, the system may include a risk monetization calculator for cyber-risk, identifying specific cyber risks, categorizing likely types of attacks, determining expected risks and costs, and breaking down company characteristics to include costs of past attacks on similar companies. Optionally, the system may include a weighting system where historical costs of similar previous attacks weigh into the calculations.

Issues taken into account in the cyber risk monetization calculator may include data breaches, leaked credentials, exposed assets, cloud misconfiguration, supply chain breaches, etc. Each issue may be assigned a weighted value. Risks monetized in the cyber risk analysis may include ransomware, IoT attacks, cloud attacks, phishing attacks, blockchain and cryptocurrency attacks, software vulnerabilities, machine learning and AI attacks, stolen funds, loss of business continuity, etc. Each risk may be assigned a weighted value.

The type of industry and geolocation of the company may also be taken into account in the monetization of cyber risk analysis, with each type and geolocation assigned a weighted value. Material costs such as investigation and recovery, as well as non-material costs like data breaches and leaked credentials, may be included in the monetization, each assigned a weighted value.

This detailed description outlines the various modules and functionalities that may be included in the cybersecurity system 160, providing a comprehensive overview of its capabilities and potential configurations.

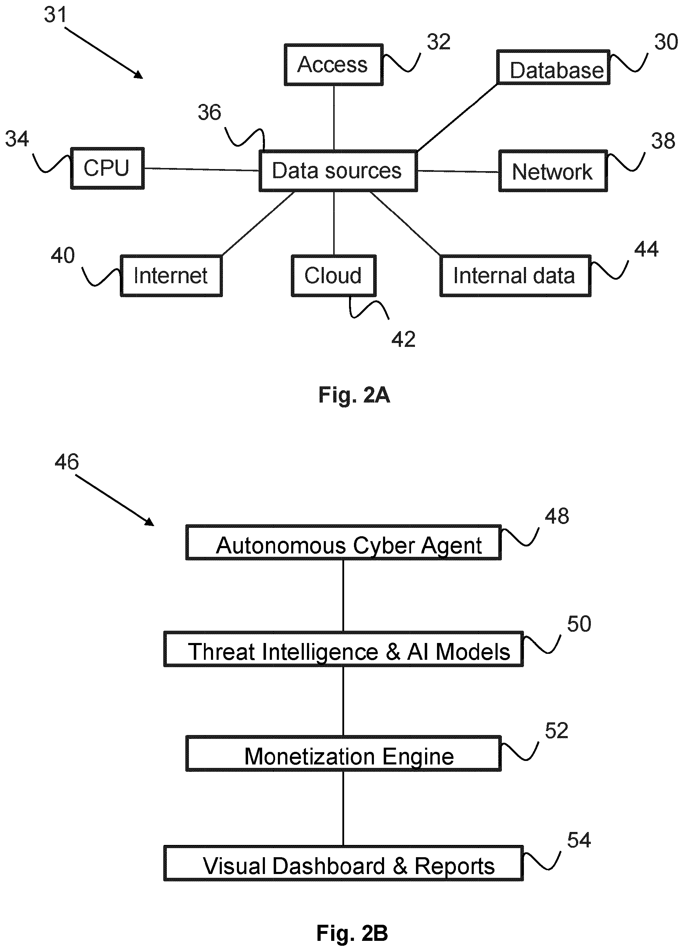

Fig. 2A is a block diagram of various data sources in accordance with some embodiments. For example, system 31 may have access 32 to data 36 from various sources on the vulnerabilities of a company may be obtained from the internet 40, cloud 42, internal data and/or external data 44, internal and/or external network 38, individual CPUs 34, data access points 32 (e.g., virtual and/or physical access), internal databases and/or external databases 30, or combinations thereof.

Fig. 2B is a block diagram of the cyber risk monetization process in accordance with some embodiments. For example, in system 46, an autonomous cyber agent 48 scans exposure across cloud, SaaS, infrastructure, threats and relevant data privacy regulations and standards. Optionally, the cyber agent 48 may take into account data such as the industry, threat, vulnerability, 3D historical cases (e.g., insurance claims, of identified vulnerabilities, reported), location (e.g., national, continent, etc.), industry (e.g., size, type, etc.).

In some embodiments, collecting risk data on cyber-attacks from an SaaS platform may involve monitoring and analyzing various types of telemetry data generated by the infrastructure that supports the service. For example, telemetry data may include information about system performance, user activity, and network traffic. The telemetry data may be used to provide insights into potential security threats. For example, logs from servers and/or applications may reveal patterns indicative of malicious activity. Example, of such patterns may include repeated failed login attempts, unusual data transfer volumes, and/or access from suspicious IP addresses. Additionally or alternatively, telemetry data can include metrics related to system health and vulnerabilities.

In some embodiments, regulatory data feeds will be used as a source of risk data for cyber-attacks. For example, a regulatory data feed may provide information on compliance requirements, threat intelligence, and/or industry standards that organizations are to adhere to e.g., in order to protect their data and systems. Regulatory data may include, for example, updates on new laws and regulations, advisories on emerging threats, and/or best practices for cybersecurity. Data may be integrated into the risk management processes automatically and/or constantly and/or in real time. The resulting up-to-date data and/or security protocols and may facilitate proactively response to a change in the regulatory landscape. This may help in maintaining compliance and/or reducing the risk of penalties and/or reducing the risk of breaches due to non-compliance.

In some embodiments, data collected from SaaS platforms and/or regulatory feeds may be diverse and/or complementary. Optionally, from a SaaS platform the cyber agent may gather detailed telemetry data including, for example, system logs, user activity reports, and/or network traffic analyses. Data may facilitate identifying and/or understanding potential vulnerabilities and/or threats within the infrastructure. Additionally or alternatively, regulatory feeds may provide contextual information for interpreting telemetry data in light of current security standards and/or compliance requirements. For example, the regulatory feeds may include threat intelligence reports, legal updates, and/or guidelines for best practices. Combining these sources of data may facilitate a comprehensive view of their cybersecurity risk landscape, enabling more effective prevention, detection, and response strategies.

In some embodiments, a threat intelligence model and AI models 50 estimate probability and/or impact based on actual threats and vulnerabilities, which may optionally include third parties and/or supply chains. A monetization engine 52 may quantify financial loss estimates based on prior cases with similar characteristics (industry, sector, size, geolocation, threat context, etc.). The monetization engine may be configured to make an assessment of expected financial loss from a specified cyber-attack to an entity classified by geolocation, industry type, and size, and/or based on risk data. Optionally, risk data may be collected from insurance claims, reported threats, reported vulnerabilities, etc. Optionally, the risk data may be further classified by demographics of involved populations, such as customers and/or employees. The results may be displayed on a virtual dashboard 54, and/or as reports for stakeholders, boards, auditors, etc.

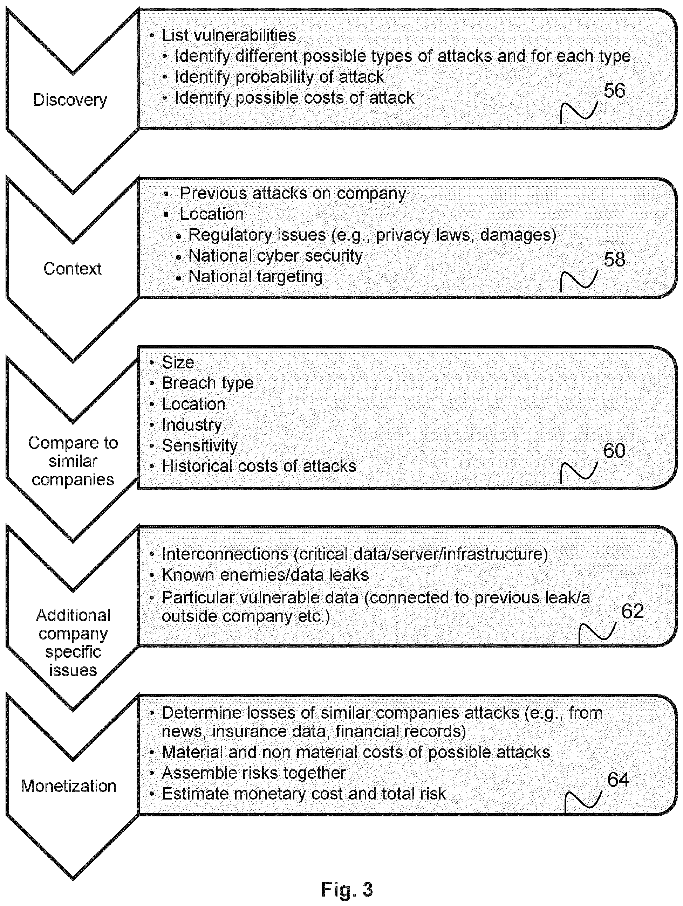

Fig. 3 is a flow diagram of the cyber risk monetization process in accordance with some embodiments. For example, the first stage may include the discovery stage 56. Optionally, during the discovery stage 56 a list of vulnerabilities may include identification, e.g., identify different possible types of attacks and for each type, identify the probability of attack, identify possible costs of an attack, etc. The second stage may include the localization stage 58, wherein assessment of local and/or company specific factors may be made, e.g., previous attacks on company, geolocation, regulatory issues (e.g., privacy laws, damages, etc.), national cyber security infrastructure and/or protection and/or mitigation, national targeting (for example, is this country under heavier or lighter attack [for example, countries with known enemies using cyber warfare against them and/or countries with large internal cybercrime may have higher risks]), etc. In the third stage, comparisons 60 to similar companies may be made, e.g., based on the size of the company, breach type, location, industry, sensitivity, historical costs of attacks on similar companies, etc. Optionally, some types of businesses and/or institutions may make them more likely to be the targets of cyber-attacks. Optionally, the sensitivity of the material and or the company and/or the industry to be protected may affect the likelihood of cyber-attacks. Additional company-specific issues 62 may also be assessed, e.g., a company's interconnections (e.g., critical data, server, infrastructure, etc.), known enemies, competitors, data leaks, etc., and particularly vulnerable data (e.g., connections to previous leaks, external companies, etc.). Optionally, additional data from national and/or international databases may be included in the assessment, e.g., data from insurance claims after cyber incidents, etc. The various risks and probabilities may be assembled together and monetized to determine a monetary risk 64. Vulnerabilities may include known weaknesses in software or hardware. The system may determine the possible types of attacks and/or their probability and/or monetize an expected and/or maximum and/or minimum likely cost of the attack. In some embodiments, the process may be cyclic. For example, the process is repeated as new vulnerabilities are detected while older ones might have been addressed already. Optionally, the process may be practiced by a monetization engine.

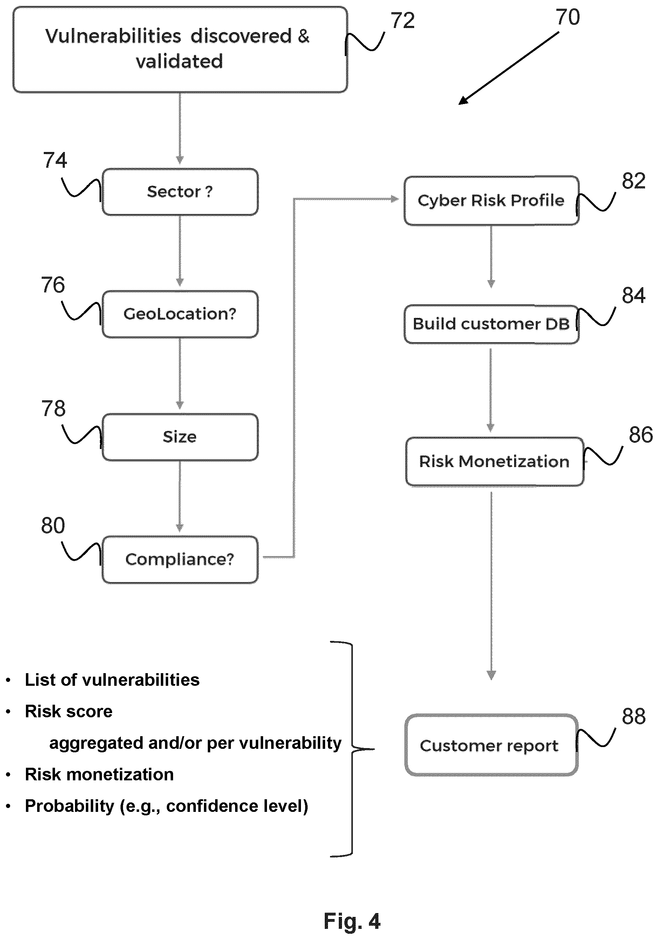

Fig. 4 is a flow diagram of a cyber risk assessment, in accordance with some embodiments. For example, in method 70, a variety of factors may be included in a monetization calculator. Optionally, each factor may be weighted. Optionally, vulnerabilities may be discovered and validated 72 by taking into account factors such as business sector 74, geolocation 76, company size 78, compliance 80 with local regulations and/or laws, etc. From this, a cyber risk profile 82 may be determined. A company database (DB) 84 may be built, and the identified risks monetized 86. A report 88 may be provided to the customer including, e.g., a list of all vulnerabilities, prioritization of such vulnerabilities, a risk score (aggregated and specific per vulnerability, risk monetization, probability (such as confidence level, etc.) for the risk, etc.

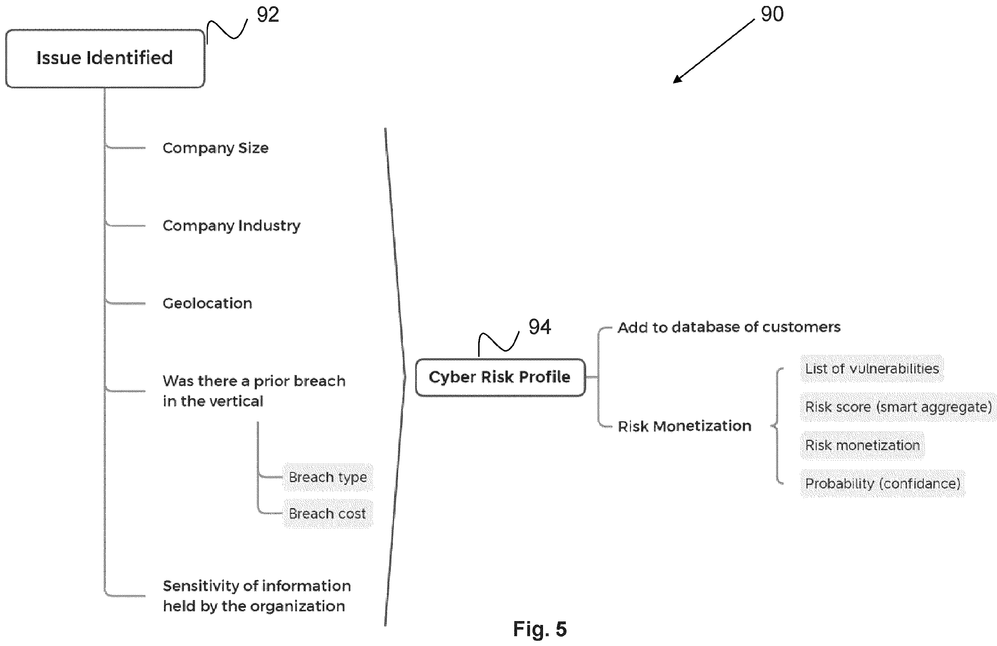

Fig. 5 is a flow diagram of a cyber risk assessment, in accordance with some embodiments. For example, in method 90, an issue may be identified 92. Factors such as company size, company industry, geolocation, prior breach (e.g., breach type, breach cost, action taken in response, etc.), sensitivity of the information held by the company, etc. A cyber risk profile may be generated 94, which may be added to a database of customers, and/or the risk may be monetized, e.g., list of vulnerabilities, risk score (smart aggregate), risk monetization, probability (e.g., confidence level), etc. Optionally, the process may be practiced by a monetization engine.

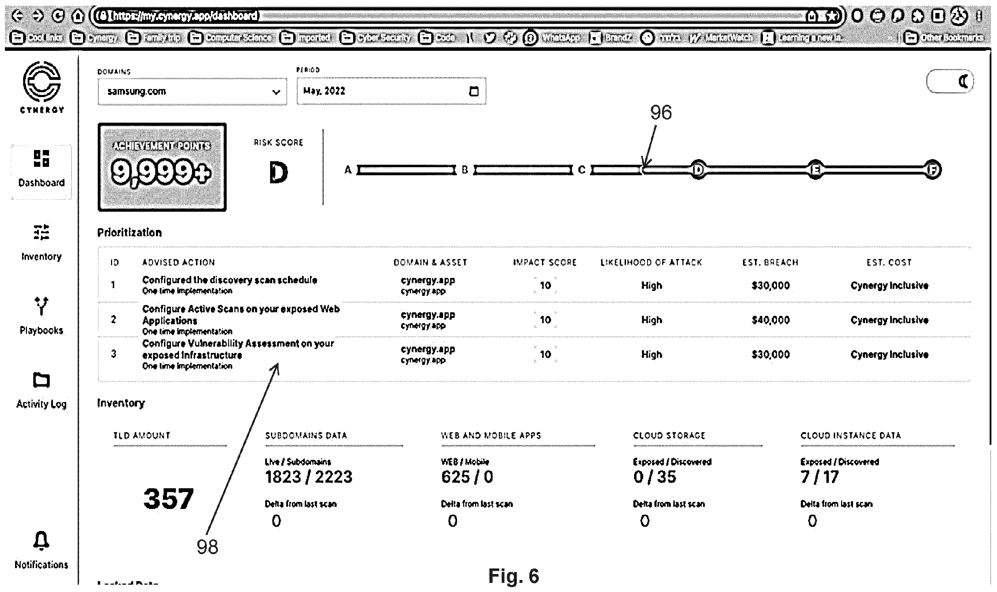

Fig. 6 is a schematic diagram illustrating cyber risk assessment dashboard output interface to a user in accordance with some embodiments. According to some embodiments, a dashboard is supplied to clarify to facilitate a user understanding a summary of costs of cyber risks to an entity and/or to acquire more information about the costs and/or risks. Optionally, the dashboard is connected to and/or supplies access to a local program and/or resources and/or to a cloud-based platform and/or resources. For example, the dashboard may display top rated issues 98 (e.g., issues combining relatively high risk and/or high cost). Optionally the system will display a cyber performance rating 96. For example, the score may facilitate the comparison of exposure with other entities and/or opportunities. In some embodiments output may include an API and/or a report.

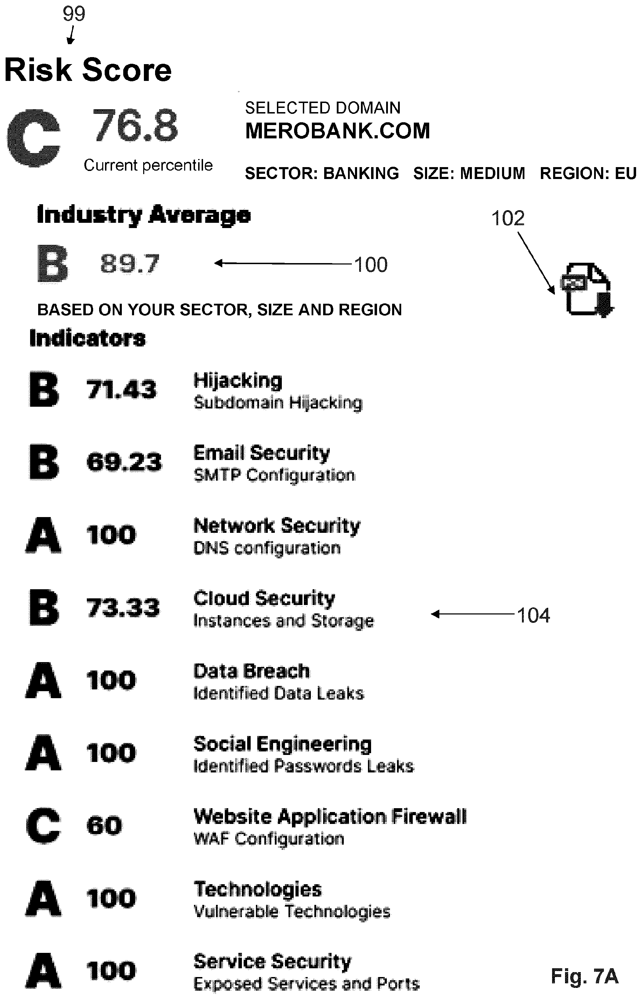

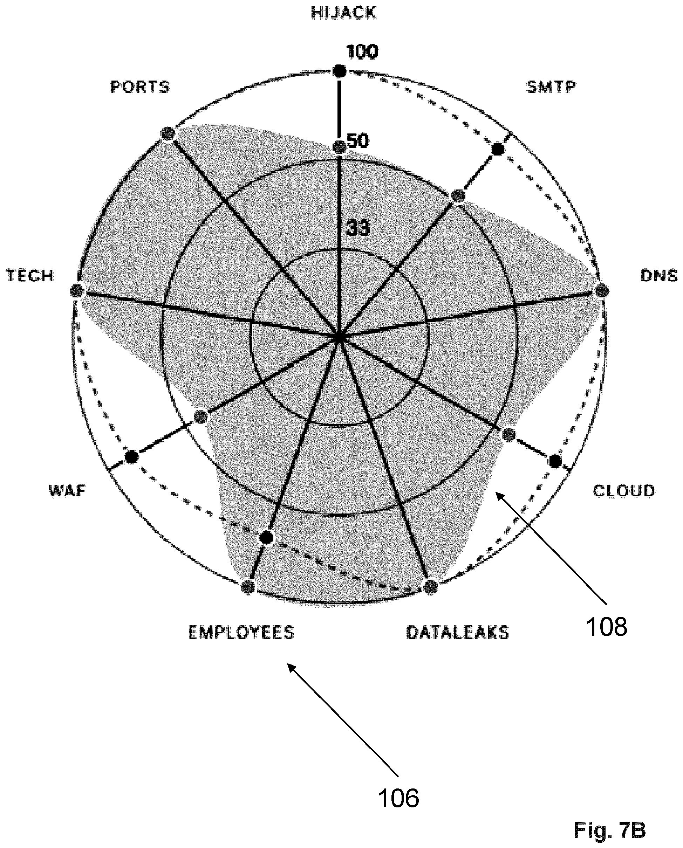

Figs. 7A and 7B are a schematic diagram illustrating cyber risk assessment risk score user output interface in accordance with some embodiments. According to some embodiments, the system may facilitate comparison with similar entities (e.g., an industry average and/or average for a similar-sized entity and/or similarly located entity). Optionally, the interface will display graphically and/or textually the distribution of monetary risks over different scenarios and/or causes. Optionally, the report may display an overall risk exposure score 99 in comparison to an industry average 100 (e.g., using a benchmark for the industry, segment, region size, etc.). Optionally, the report may include a breakdown of the score 104. The system may include many kinds of costs and/or attacks. For example, costs may include repair of damage, legal costs, loss of business, losses due to interruptions of service, losses due to loss of data, losses of completeness, loss of market, damage to reputation etc. Various factors 106 may be shown graphically and compared to the benchmark 108. Optionally, the report may be downloadable 102.

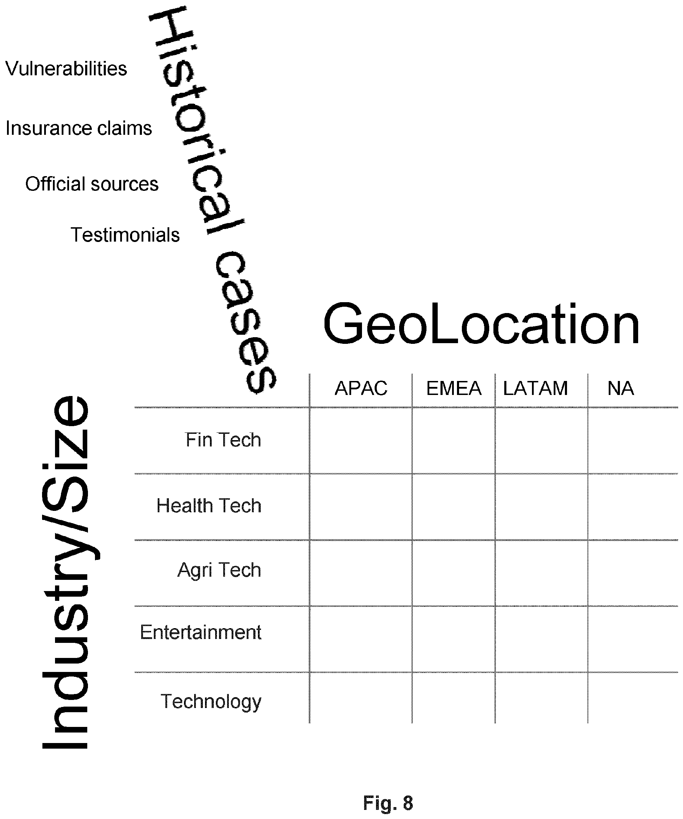

Fig. 8 is a schematic diagram illustrating a database in accordance with some embodiments. A database may include data from various data sources of the costs associated with cyber issues. Optionally, the process may be practiced by a monetization engine.

For example, data may include data on vulnerabilities and risk assessments and/or on insurance claims and/or cost data from official sources and/or testimonial data on monetary losses etc. Optionally the granularity/detail of the categorization may be data dependent. For example, in regions and/or industries where more data is available, smaller regions and/or more precise categories may be defined. Optionally, data may be weighted. For example, monetization of a risk may account for a very close size businesses and/or very close type industries and/or very close geological locations at a high weight and/or monetization of a risk may account for different size businesses and/or different type industries and/or different geological locations at a lower weight. Optionally data may be aggregated. For example, geographical regions with similar laws, conditions and/or cyber costs may be aggregated even when they are physically far apart. Similarly, industry data may be aggregated. Optionally, data may be disaggregated. For example, the effect of correlation between business type and location may be disaggregated from the data. For example, the effect of correlation between business type and size may be disaggregated from the data. Disaggregation may be used to get a more accurate measure of the importance of various factors and/or more accurate monetization of risks.

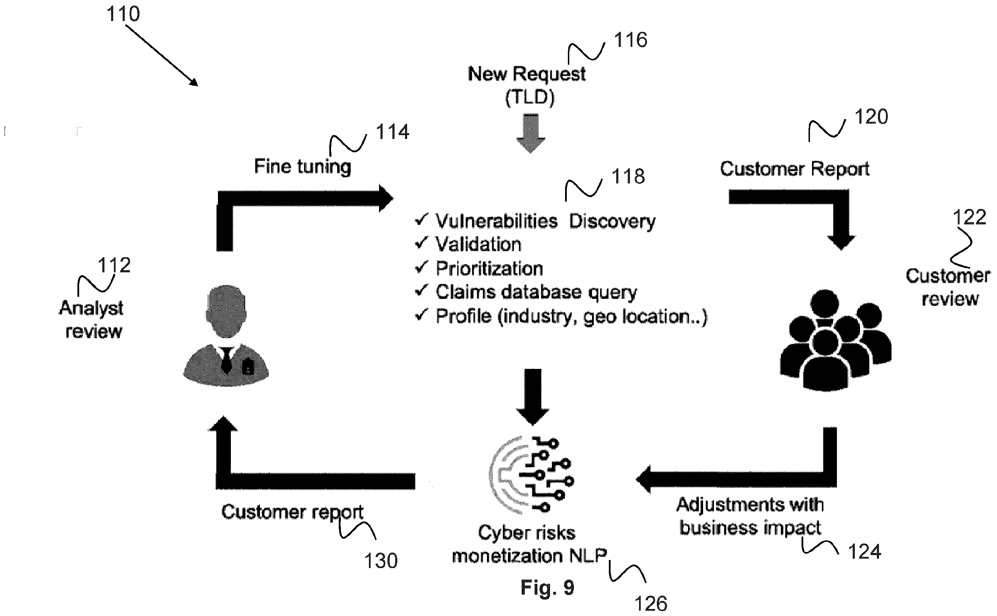

Fig. 9 is a flow diagram of a cyber risk assessment product process flow, in accordance with some embodiments. For example, in method 110, a new top-level domain (TLD) request is received 116 by the system and is analyzed 118 according to a process described herein, e.g., vulnerability discovery, validation, prioritization, claims database query, profile (industry, geolocation, etc.). Optionally, a customer report may be generated 120 for customer review 122. After the customer makes adjustments to improve cyber security, the adjustments impact on the business may be reassessed 124 and/or cyber risks may be monetized 126. Alternatively, or additionally, after the system has been analyzed 118, the cyber risks may be monetized 126 and a customer report may be generated 130, which may undergo analyst review 112 and/or fine tuning 114. Optionally, the analyst may include a human analyst and/or an AI model. Optionally, the process may be repeated and/or iterated for optimization and to ensure all vulnerabilities have been discovered, validated, prioritized, and then resolved and/or monetized. Optionally, the process may be practiced by a monetization engine.

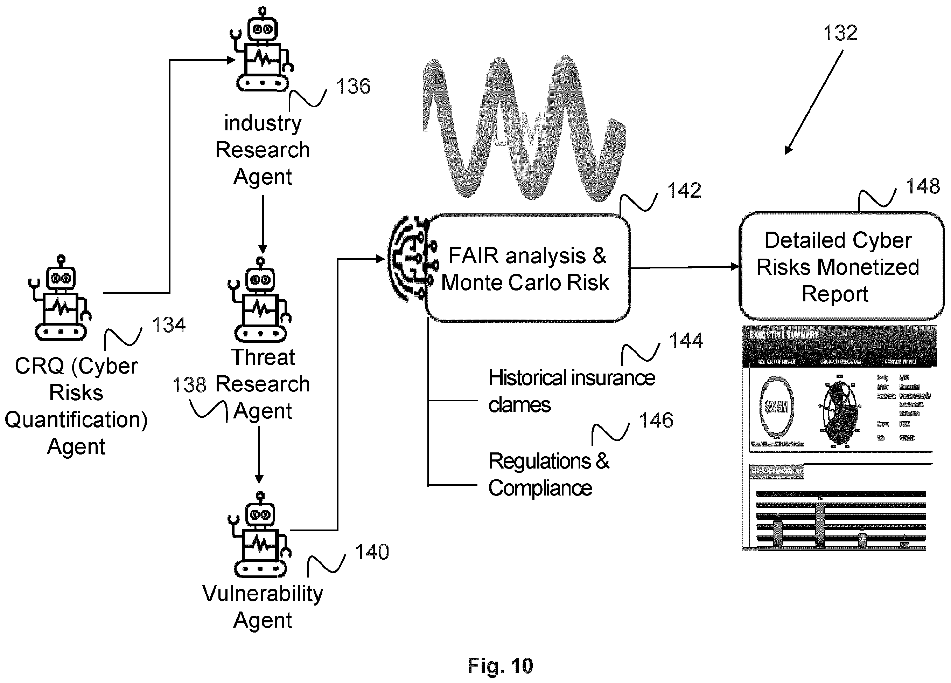

Fig. 10 is a flow diagram of a cyber risk assessment, in accordance with some embodiments. For example, in method 132, a number of AI models may be used to assess and/or monetize cyber risk. Some of these AI models may include a cyber risk quantification agent (CRQ) 134 configured to discover vulnerabilities, and/or validate the data, and/or prioritize the discovered vulnerabilities. An industry research agent 136 model may be a model specific to a particular industry and/or geolocation and/or business segment and/or business size, etc. Optionally, the industry research agent 136 model may collect and/or assess and/or add industry specific information to the data from the cyber risk quantification agent 134. A threat research agent 138 model may collect and/or assess and/or add up to date threat data to the data from the cyber risk quantification agent 134 and/or industry research agent 136. A vulnerability agent 140 model may be configured to discover client specific vulnerabilities. Optionally, data from the vulnerability agent 140 may be added to data from the cyber risk quantification agent 134 and/or industry research agent 136 and/or threat research agent 138. Optionally, Ai model (e.g., a large language model (LLM)) 142 may perform analysis on the collected and/or generated data, e.g., FAIR analysis, and/or Monte Carlo risk analysis, etc. Alternatively or additionally, other Ai model may be used for analysis, for example a small language model, a rule-based system, a machine learning model, a deep learning model (e.g., Convolutional Neural Networks (CNNs) and Recurrent Neural Networks (RNNs), or transformer models), vector search, semantic search and/or a narrow language model. Optionally, historical insurance claims 144 and/or regulations and compliance data 146 may be added to the data analyzed by the large language model 142. A detailed cyber risk monetized report 148 may be generated. Optionally, the process may be practiced by a monetization engine.

In some embodiments, an Ai model (e.g., Large Language Model (LLMs)) may be used in FAIR (Factor Analysis of Information Risk) analysis. For example, in the initial phase of information gathering and contextualization, Ai model may be used to process unstructured data, such as incident reports, vulnerability scans, audit findings, policy documents, threat intelligence feeds, industry reports, news articles, internal communications, and human expert interview transcripts. By extracting relevant information about assets, threats, vulnerabilities, and controls, Ai model may optionally provide a more comprehensive view of the organization's security posture. For example, this may facilitate a more thorough and accurate risk assessment.

In some embodiments, in the phase of entity recognition and relationship extraction, an LLM may be used to identify and/or categorize entities within an organization's ecosystem. For example, this may include specific systems, data types, threat actors, and/or security controls. Optionally, the AI model will facilitate understanding the relationships between these entities and/or the AI models may be used to map out the intricate web of interactions and dependencies that influence the organization's risk profile. Optionally, this mapping may be used for understanding how different elements contribute to potential security incidents and/or how they can be mitigated. Furthermore, AI models may be used interpret the nuances of security incidents. For example, the AI model may be used to understand the business context of assets. Additionally or alternatively, the AI model may be used to categorize information according to FAIR's taxonomy, such as distinguishing between Threat Event Frequency and Vulnerability factors.

In some embodiments, in quantitative factor estimation, an AI models may be used to provide initial estimates based on the ingested data. For example, the AI model may suggest ranges or point estimates for FAIR factors. Optionally, the FAIR factors estimated by the AI model may include estimating threat event frequency (e.g., indicate how often a specific threat event might occur). Additionally or alternatively, the FAIR factors estimated by the AI model may include estimating vulnerability (e.g., the likelihood of control failure). Additionally or alternatively, the FAIR factors estimated by the AI model may include estimating loss magnitude (e.g., the cost of a data breach, downtime and/or fines). Optionally, estimates may be derived through pattern recognition from historical data and/or reasoning based on contextual information. Additionally or alternatively, an AI model may assist in scenario generation. For example, the AI model may define and/or articulate specific loss scenarios. Defining a loss scenario may include, for example, combining identified threats, assets, and vulnerabilities.

In some embodiments, an AI model may contribute to generating loss distributions conceptually. For example, an AI model may be used to interpret the results of such a Monte Carlo simulation generated by another systems. Optionally, the AI model may structure the input parameters for Monte Carlo simulations. For example, the structuring may be based on qualitative data. The AI model structure may facilitate grounding the simulations in accurate and/or relevant information. Additionally or alternatively, an AI models may perform sanity checks and/or plausibility assessments by identifying inconsistencies and/or implausible estimates generated by humans and/or other systems. For example, the AI model may compare estimates against general knowledge and/or industry benchmarks on which the AI model was trained.

These embodiments are provided by way of example and are in no means intended to limit the scope of the invention.

While the invention has been described in its preferred form or embodiment with some degree of particularity, it is understood that this description has been given only by way of example and that numerous changes in the details of construction, fabrication, and use, including the combination and arrangement of parts, may be made without departing from the spirit and scope of the invention.

In some embodiments, an AI model may be used for data normalization. The AI model may, for example, standardize disparate data formats and structures to create a cohesive dataset for analysis. Optionally, an AI model may streamline this process by intelligently parsing and transforming diverse data sources into a unified format. Advanced natural language processing capabilities of an AI models may be used to identify and/or correct inconsistencies, fill in missing information, and/or standardize terminologies. This may assist in acquiring accurate data that is ready for further analysis.

In some embodiments, an AI model will be used for entity context enrichment. For example, an AI models may be used to analyze data from various entities such as users, devices, and network nodes that interact. The AI model may be used to enrich the context around each entity. For example, the AI model may identifying relationships, behaviors, and attributes that might not be immediately apparent. In some cases, AI models may detect anomalous patterns and/or suspicious activities. Identifying may include defining the typical behavior of an entity within a network and/or recognizing deviations.

### GENERAL

It is expected that during the life of a patent maturing from this application many relevant building technologies, artificial intelligence methodologies, computer user interfaces, image capture, and devices will be developed and the scope of the terms for design elements, analysis routines, user devices is intended to include all such new technologies *a priori.*

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, some embodiments of the present invention may be embodied as a system, method or computer program invention. Accordingly, some embodiments of the present invention may take the form of an entire hardware embodiment, an entire software embodiment (including firmware, resident software, micro-code, etc.) or an embodiment combining software and hardware aspects that may all generally be referred to herein as a "circuit," "module" or "system." Furthermore, some embodiments of the present invention may take the form of a computer program product embodied in one or more computer readable medium(s) having computer readable program code embodied thereon. Implementation of the method and/or system of some embodiments of the invention can involve performing and/or completing selected tasks manually, automatically, or a combination thereof. Moreover, according to actual instrumentation and equipment of some embodiments of the method and/or system of the invention, several selected tasks could be implemented by hardware, by software or by firmware and/or by a combination thereof, e.g., using an operating system.

For example, hardware for performing selected tasks according to some embodiments of the invention could be implemented as a chip or a circuit. As software, selected tasks according to some embodiments of the invention could be implemented as a plurality of software instructions being executed by a computer using any suitable operating system. In an exemplary embodiment of the invention, one or more tasks according to some exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments of the invention. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, and/or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present invention may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present invention may be described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Data and/or program code may be accessed and/or shared over a network, for example the Internet. For example, data may be shared and/or accessed using a social network. A processor may include remote processing capabilities for example, available over a network (e.g., the Internet). For example, resources may be accessed via cloud computing.

The terms "cloud computing" and "the cloud" are used interchangeably and may refer to the use of computational resources that are available remotely over a public network, such as the internet, and which may may be searched for data. Any virtual or physical computer that is in electronic communication with such a public network could potentially be available as a computational resource. The cloud may provide computational resources via a remote network on a secure basis, computers **that access** the cloud network may employ standard security encryption protocols such as SSL and PGP, which are well known in the industry.

Some of the methods described herein are generally designed only for use by a computer and may not be feasible or practical for performing purely manually, by a human expert. A human expert who wanted to manually perform similar tasks might be expected to use completely different methods, e.g., making use of expert knowledge and/or the pattern recognition capabilities of the human brain, which would be vastly more efficient than manually going through the steps of the methods described herein.

As used herein the term "about" refers to ± 10%

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. **In** addition, citation or identification of any reference in this application shall not be construed as an admission that such reference is available as prior art to the present invention. To the extent that section headings are used, they should not be construed as necessarily limiting.

## Claims

1. A system for evaluating cyber risk of an entity comprising:
a risk evaluation module for collecting
risk data on cyber-attacks from multiple sources including SaaS platforms, infrastructure telemetry, and regulatory data feeds and
an entity evaluation module for identifying vulnerability data on assets of the entity classified by industry type, geolocation, size and cyber threat vector vulnerabilities;
a structured data classification module that organizes the collected risk data by geolocation, industry type, and size of a victim organization; and
a trained machine learning monetization engine configured for:
receiving the classified risk data,
receiving the vulnerability data,
correlating the risk data and the vulnerability data;
correlating the classified risk data with historical cyber incident datasets and insurance claims and
generates a probability-adjusted cyber incident profile comprising estimated financial loss values and incident likelihoods for an entity.

2. The system of claim 1, wherein said risk evaluation module includes cyber agent executable on a cloud-based computing infrastructure and configured to function autonomously.

3. The system of claim 1, wherein the structured data classification module and monetization engine are configured to update assessments dynamically in response to new risk data supplied by the risk evaluation module.

4. The system of claim 1, further comprising:
an AI threat assessment module configured to estimate an probability of a specified cyber-attack on the entity based on said identified threats, and said identified vulnerabilities.

5. The system of claim 4, wherein said AI threat assessment module is further configured to estimate an impact of said specified cyber-attack based said risk data.

6. They system of any one of claims 1, 4 and 5, further comprising a user-facing analytics engine comprising a dashboard interface configured to present the incident profile in visual, board-level decision support format.

7. The system of claim 1, wherein the financial loss values and incident likelihoods includes social engineering data relating to identification of employees, password leaks, or phishing.

8. A computer-implemented method for cyber risk quantification for an entity comprising:
collecting cyber risk data from at least one of a SaaS system, an infrastructure log, a legal database and a regulatory databases;
classifying the risk data by location, industry type, and size of a victim organization; identifying vulnerabilities for the entity; and
estimating a probability of a specified cyber-attack by correlating said risk data and said vulnerabilities.

9. The method of claim 8, further comprising executing a predictive monetization model to calculate an expected financial loss from a specified cyber-attack to an entity classified by location, industry type, and size based on the collected risk data using a predictive monetization engine.

10. The method of claim 9, further comprising:
training the predictive monetization model using:
historical cyber insurance claim data;
previously reported vulnerability disclosures; and
confirmed threat intelligence indicators.

11. The method of claim 9, further comprising:
Automatically retraining the predictive monetization model periodically using:
historical cyber insurance claim data;
previously reported vulnerability disclosures; and
confirmed threat intelligence indicators.

12. The method of claim 9, further comprising:
estimating an impact of said specified cyber-attack based on said identified threats and an identified vulnerabilities using the predictive monetization model.

13. The method of claim 9, further comprising:
collecting said risk data from insurance claims of reported threats and reported vulnerabilities.

14. The method of claim 8, wherein said classifying is further by demographics of involved populations.

15. The method of any one of claims 10, 12, 13, and 14, further comprising:
presenting an assessment of a plurality of said expected financial losses to at least one of stakeholders, boards, and auditors using a visual dashboard and reporting module.
